# EUROPEAN PATENT APPLICATION

(11) **EP 4 403 186 A1**
(43) Date of publication of application: **24.07.2024**
(21) Application number: 22870048.0
(22) Date of filing: 16.09.2022
(51) Int. Cl.: A61K 45/00, A61P 11/00, A61P 35/00, C12N 5/09, C12N 1/00, G01N 33/574, A61K 31/4985

(54) **PRODUCTION OF ORGANOID DERIVED FROM CTC OF SMALL CELL LUNG CANCER PATIENT, AND IGF1R INHIBITOR AS A MOLECULAR TARGET DRUG FOR SMALL CELL LUNG CANCER**

(30) Priority: 16.09.2021 JP 2021151032
(71) Applicant: Keio University, Tokyo, 108-8345 (JP)
(72) Inventor: YASUDA Hiroyuki, Tokyo 160-8582 (JP); SATO Toshiro, Tokyo 160-8582 (JP); EBISUDANI Toshiki, Tokyo 160-8582 (JP); FUKUSHIMA Takahiro, Tokyo 160-8582 (JP); FUKUNAGA Koichi, Tokyo 160-8582 (JP); HAMAMOTO Junko, Tokyo 160-8582 (JP)
(74) Representative: Cabinet Becker et Associés
(86) International application number: PCT/JP2022/034701
(87) International publication number: WO 2023/042901

(57) **Abstract**

A therapeutic agent for small cell lung cancer containing an insulin-like growth factor 1 receptor (IGF1R) inhibitor as an active ingredient, and a method whether or not an administration of the therapeutic agent for small cell lung cancer patients is effective, including: a step of detecting the expression of YAP1 or its downstream factors in the cancer cells from the small cell lung cancer patient; and a step of detecting the expression of YAP1 or its downstream factors in the cancer cells from the small cell lung cancer patient, wherein when the expression level of YAP1 or the expression level of the downstream factor of YAP1 is significantly higher than that of a control, the administration of the therapeutic agent is effective in treating the patient with small cell lung cancer.

## Description

### [Technical Field]

The present invention provides that insulin-like growth factor 1 receptor (1GF1R) inhibitors are effective against YAP1-positive small cell lung cancer, which is one of the small cell lung cancer subtypes. The present invention also provides a method for determining whether administration of an IGF1R inhibitor is effective in treating small cell lung cancer patients. In addition, the present invention relates to a method for producing small cell lung cancer cell-derived organoids and a method for immunostaining YAP1 protein. This application claims priority based on Japanese Patent Application No. 2021-151032 filed in Japan on September 16, 2021, the contents of which are incorporated herein.

### [Background Art]

In recent years, molecular target therapeutic agents targeting specific genes have been developed in the field of cancer, and multiple drugs have improved the prognosis of cancer patients.

Small cell lung cancer is a disease with a poor prognosis, accounting for approximately 15% of all lung cancers. Small cell lung cancer is a rapidly progressing cancer, and in most cases, it is already inoperable at the time of diagnosis. For this reason, it has been difficult to collect a sufficient amount of patient-derived specimens, making it difficult to analyze their biological properties. In the field of small cell lung cancer, no treatment method using molecular target therapeutic agents has been developed to date, and patient prognosis has not improved.

In recent years, it has been reported that small cell lung cancer can be classified into subtypes based on the expression of transcription factors ASCL1, NEUROD1, POU2F3, and YAP1 (see Non-Patent Document 1).

### [Citation List]

### [Non-Patent Documents]

Baine M. K., et al., SCLC Subtypes Defined by ASCL1, NEUROD1, POU2F3, and YAP1: A Comprehensive Immunohistochemical and Histopathologic Characterization, Journal of Thoracic Oncology, 15 (12), 1823-1835, 2020.

### [Summary of Invention]

### [Technical Problem]

In order to improve the prognosis of patients with small cell lung cancer by developing treatments using molecular target therapeutic agents, it is necessary to identify patient subgroups for which molecular target therapeutic agents are effective, and to develop effective treatments for those subgroups. However, until now, it has not been known whether there are subgroups for which treatment with molecular target therapeutic agent is effective, which molecular target therapeutic agents are effective for these subgroups, and whether there is a method for determining the effectiveness of administration of molecular target drugs.

The object of the present invention is to provide a technology for identifying a subgroup of small cell lung cancer for which administration of a molecular target drug is effective, elucidating the molecular target drug, and determining the effectiveness of administering the molecular target drug.

### [Solution to Problem]

The present invention includes the following aspects.
[1] A therapeutic agent for small cell lung cancer, which contains an insulin-like growth factor 1 receptor (IGF1R) inhibitor as an active ingredient.
[2] The therapeutic agent according to [1], wherein the small cell lung cancer is a small cell lung cancer in which an expression level of Yes1 Associated Transcriptional Regulator (YAP1) or an expression level of a downstream factor of YAP1 is significantly higher than that of a control.
[3] The therapeutic agent according to [2], wherein the downstream factor of YAP1 is connected tissue growth factor (CTGF), cysteine-rich angiogenic inducer 61 (CYR61), or Cyclin D1 (CCND1).
[4] A method for determining whether or not an administration of the therapeutic agent according to any one of [1] to [3] is effective in treating a small cell lung cancer patient, including:
   a step of detecting an expression level of YAP 1 or an expression level of a downstream factor of YAP1 in a cancer cell from the small cell lung cancer patient,
   wherein when the expression level of YAP1 or the expression level of the downstream factor of YAP1 is significantly higher than that of a control, it is shown that the administration of the therapeutic agent according to any one of [1] to [3] is effective.
[5] The method according to [4], wherein the downstream factor of YAP1 is CTGF, CYR61 or CCND1.
[6] The method according to [4], wherein the detection of the expression of YAP1 is performed by immunostaining of YAP1 protein.
[7] The method according to any one of [4] to [6], wherein the cancer cell from the patient with small cell lung cancer is a small cell lung cancer cell-derived organoid.
[8] A method for producing a small cell lung cancer cell-derived organoid, including a step of culturing a cancer cell obtained from a small cell lung cancer patient in a medium.
[9] The method for producing according to [8], wherein the medium contains: at least two selected from the group consisting of insulin-like growth factor 1 (IGF1), fibroblast growth factor 2 (FGF2), EGF (Epidermal Growth Factor), and epiregulin (EREG); and at least one selected from the group consisting of Wnt agonists, bone morphogenetic protein (BMP) inhibitors, and transforming growth factor-β (TGF-β) inhibitors.
[10] The method for producing according to [8] or [9], wherein the cancer cell is a cancer tissue-derived cancer cell, a pleural effusion-derived cancer cell, a sputum-derived cancer cell, or a circulating cancer cell.

### [Advantageous Effects of Invention]

According to the present invention, a technique can be provided for identifying a subgroup of small cell lung cancer for which administration of a molecular target therapeutic agent is effective, clarifying the molecular target therapeutic agent, and determining the effectiveness of administration of the molecular target therapeutic agent.

### [Brief Description of Drawings]

FIG. 1 is a heat map created based on the results of RNA-seq analysis in Experimental Example 2.
FIG. 2 is a graph showing the results of principal component analysis based on the results of RNA-seq analysis in Experimental Example 2.
FIG. 3 is a micrograph of a representative small cell lung cancer organoid in Experimental Example 3.
FIG. 4 is a diagram showing the relationship between IGF dependence, types of transcription factors expressed, and clusters of small cell lung cancer organoid lines in Experimental Example 3.
FIG. 5 is a diagram showing the results of co-expression cluster analysis of IGF-dependent organoids and IGF-independent organoids in Experimental Example 3.
FIG. 6 is a diagram showing the results of hierarchical cluster analysis on IGF-dependent organoids and IGF-independent organoids in Experimental Example 3.
FIG. 7 is a photograph showing the results of immunostaining of small cell lung cancer organoids in Experimental Example 4.
FIG. 8 is a graph showing the measurement results of the number of living cells in Experimental Example 5.
FIG. 9 is a representative photograph of small cell lung cancer organoids 5 days after addition of 1 µM linsitinib in Experimental Example 5.
FIG. 10 is a graph showing the results of flow cytometry in Experimental Example 6.
FIG. 11 is a graph showing the results of measuring tumor volume over time in Experimental Example 7.

### [Description of Embodiments]

As described later in Examples, the inventors have invented a method for producing small cell lung cancer cell-derived organoids, which includes a step of culturing cancer cells derived from a small cell lung cancer patient in a medium. In addition to small cell lung cancer cell organoids expressing POU2F3, it was also possible to produce small cell lung cancer cell organoids with a high expression level of YAP1 or its downstream factors. The inventors also revealed that small cell lung cancer cells which express a high level of YAP1 or its downstream factors require IGF1 for their proliferation, that is, that are IGF dependent.

The inventors further revealed that small cell lung cancer cells that express a high level of YAP1 or its downstream factors have suppressed proliferation and induced apoptosis in the presence of an 1GF1R inhibitor.

Therefore, administration of an IGF1R inhibitor is effective in treating small cell lung cancer patients with a high expression level of YAP1 or its downstream factors. A subgroup of small cell lung cancer cells for which administration of these IGF1R inhibitors is effective is small cell lung cancer cells with high expression of the YAP1 gene, which has been previously reported in Non-Patent Document 1, and the lide, and is also called the YAP1 positive subtype. The inventors further clarified a method for determining whether administration of an IGF1R inhibitor is effective in treating patients with small cell lung cancer, and completed the present invention.

### [Method for producing organoids from small cell lung cancer cell]

In one embodiment, the present invention provides a method for producing small cell lung cancer cell-derived organoids, which includes the step of culturing cancer cells derived from a small cell lung cancer patient in a medium. An organoid is a three-dimensional cell tissue that is self-organized by an accumulation of cells, and has a structure and function similar to an organ in a living body.

Examples of cancer cells derived from small cell lung cancer patients include cancer tissue-derived cancer cells, pleural effusion-derived cancer cells, sputum-derived cancer cells, and circulating cancer cells.

In the manufacturing method of this embodiment, a preferable medium for culturing cancer cells derived from a small cell lung cancer patient is a basic medium containing at least two selected from the group consisting of insulin-like growth factor 1 (IGF1), fibroblast growth factor 2 (FGF2), Epidermal Growth Factor (EGF) and epiregulin (EREG), and at least one selected from the group consisting of a Wnt agonist, a bone morphogenetic protein (BMP) inhibitor and a transforming growth factor-β (TGF-β) inhibitor.

The culture medium for cancer cells derived from small cell lung cancer patients preferably contains at least two selected from the group consisting of IGF1, FGF2, and EGF. The medium preferably contains IGF1, more preferably FGF2 and EGF.

The culture medium for cancer cells derived from small cell lung cancer patients contains at least one selected from the group consisting of Wnt agonists, BMP inhibitors, and TGF-β inhibitors. Here, although a Wnt agonist is not essential, it is preferable to include a BMP inhibitor and a TGF-β inhibitor.

Furthermore, the culture medium for cancer cells derived from patients with small cell lung cancer may contain serum, but is preferably serum-free.

As the basic medium, any serum-free cell culture basic medium can be used. For example, a defined synthetic medium buffered with a carbonate-based buffer to a pH of 7.2 or more and 7.6 or less may be used. More specifically, Advanced Dulbecco's modified Eagle supplemented with glutamine, insulin, B27 supplement (Thermo Fisher Scientific), N-acetyl-L-cysteine (Fujifilm Wako Pure Chemical), penicillin, streptomycin, transferrin, etc. Examples include medium/Ham's F-12 mixed medium (DMEM/F12). Moreover, RPMI1640 medium, advanced RPMI medium, etc. may be used instead of DMEM/F12 medium.

### (IGF1)

IGF1 is also called somatomedin C and is a factor that is secreted mainly in the liver by stimulation by growth hormone (GH). It is known that almost all cells in a human body (particularly, cells in muscles, bones, liver, kidney, nerve, skin, lung, and the like) are affected by IGF1. In addition to an insulin-like effect, IGF1 has a function of regulating the growth and development of cells (particularly nerve cells) and cellular DNA synthesis.

The concentration of IGF1 contained in the culture medium is not particularly limited, but is preferably 5 ng/mL or higher and 1 µg/mL or lower, more preferably 10 ng/mL or higher and 1 µg/mL or lower, and still more preferably 50 ng/mL or higher and 500 ng/mL or lower.

### (FGF2)

FGF2 is a basic fibroblast growth factor, binds to a fibroblast growth factor receptor (FGFR), and has a function of the growth promotion of vascular endothelial cells and the organization into a tubular structure, that is, the promotion of the new birth of blood vessels. In addition, it is known that human FGF2 has 2 isoforms of a low-molecular-weight type (LWL) and a high-molecular-weight type (HWL). LWL is present mainly in cytoplasm and acts by autocrine, whereas HWL is present in nuclei and exhibits activity by an intracrine mechanism that acts in cells.

The concentration of FGF2 contained in the culture medium is not particularly limited, but is preferably 5 ng/mL or higher and 1 µg/mL or lower, more preferably 10 ng/mL or higher and 1 µg/mL or lower, and still more preferably 50 ng/mL or higher and 500 ng/mL or lower.

### (EGF)

EGF is a potent mitogenic factor for various cultured ectodermal cells and mesodermal cells and has a significant influence on the differentiation of some fibroblasts into specific cells. An EGF precursor is present as a membrane-bound molecule that is proteolytically cleaved to produce 53-amino acid peptide hormone that stimulates cells.

The concentration of EGF contained in the culture medium is preferably 5 ng/mL or higher and 500 ng/mL or lower, more preferably 10 ng/mL or higher and 400 ng/mL or lower, and still more preferably 50 ng/mL or higher and 200 ng/mL, or lower.

### (Wnt agonist)

Wnt agonist means a drug that activates T-cell factor (hereinafter also referred to as TCF)/lymphoid enhancer factor (hereinafter also referred to as LEF)-mediated transcription in cells. Accordingly, Wnt agonists are not limited to Wnt family proteins, but include Wnt agonists that bind to and activate Frizzled receptor family members, inhibitors of intracellular β-catenin degradation, and activators of TCF/LEF. The Wnt agonist is preferably at least one selected from the group consisting of Wnt protein, R-spondin, and GSK-3β inhibitor.

The medium for producing epithelial organoids preferably contains a Wnt agonist. The Wnt agonist more preferably includes a complex of Wnt protein and its stabilizer, afamin, and even more preferably includes a complex of Wnt protein and afamin, and R-spondin. By containing the complex of Wnt protein and afamin and R-spondin in the medium, epithelial organoids can be formed with higher efficiency.

### <<Wnt protein>>

The origin of the Wnt protein is not particularly limited, and Wnt proteins derived from various organisms can be used. Among these, Wnt proteins derived from mammals are preferred. Examples of mammals include humans, mice, rats, cows, pigs, rabbits, and the like. Examples of mammalian Wnt proteins include Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, Wnt10a, Wnt10b, Wnt11, Wnt16, etc. Can be mentioned. In the culture medium for producing epithelial organoids, a combination of a plurality of types of Wnt proteins may be used.

As a method for preparing the Wnt protein, for example, a method in which the Wnt protein is prepared using a Wnt protein-expressing cell and the like are exemplary examples. In the Wnt protein-expressing cell, where the cell is derived (organism species, culture morphology, or the like) is not particularly limited and may be a cell that stably expresses the Wnt protein or may be a cell that transiently expresses the Wnt protein. As the Wnt protein-expressing cell, for example, an L cell that stably expresses mouse Wnt3a (ATCC CRL-2647), an L cell that stably expresses mouse WntSa (ATCC CRL-2814), and the like are exemplary examples. In addition, the Wnt protein-expressing cell can be made using a well-known gene recombination technique. That is, the Wnt protein-expressing cell can be made by inserting a DNA that encodes a desired Wnt protein into a well-known expression vector and introducing the obtained expression vector into an appropriate host cell. The nucleotide sequence of a gene that encodes the desired Wnt protein can be acquired from a well-known nucleotide sequence database such as GenBank (R).

The Wnt protein that is expressed by the Wnt protein-expressing cell may be a fragment of the Wnt protein or may be a Wnt protein containing an amino acid sequence other than the amino acid sequence of the Wnt protein as long as the Wnt protein has a Wnt activity. The amino acid sequence other than the amino acid sequence of the Wnt protein is not particularly limited, and examples thereof include an amino acid sequence of an affinity tag and the like. In addition, the amino acid sequence of the Wnt protein does not need to fully coincide with an amino acid sequence that can be acquired from a well-known database such as GenBank (R) and may be an amino acid sequence that is substantially the same as an amino acid sequence that can be acquired from a well-known database as long as the amino acid sequence has a Wnt activity.

As the amino acid sequence that is substantially the same as an amino acid sequence of the Wnt protein that can be acquired from a well-known database such as GenBank (R), for example, amino acid sequences that can be acquired from a well-known database in which one to several amino acids are deficient, substituted or added and the like are exemplary examples.

The amino acid sequence in which one to several amino acids are deficient, substituted, or added means that, for example, as many amino acids as can be made deficient, substituted, or added by a well-known mutant peptide making method such as a site-directed mutagenesis method (10 or less is preferable, 7 or less is more preferable, and 6 or less is still more preferable) are deficient, substituted or added.

In addition, as the amino acid sequence that is substantially the same, for example, an amino acid sequence of which the identity to an amino acid sequence that can be acquired from a well-known database is at least 80% or more, preferably at least 85% or more, more preferably at least 90% or more, still more preferably at least 92% or more, particularly preferably at least 95% or more, and most preferably at least 99% or more or the like is an exemplary example.

The concentration of the Wnt protein is preferably 50 ng/mL or more, more preferably 100 ng/mL or more and 10 µg/mL or less, still more preferably 200 ng/mL or more and 1 µg/mL or less, and particularly preferably 300 ng/mL or more and 1 µg/mL or less.

### <<R-spondin>>

As the R-spondin, an R-spondin family consisting of R-spondin 1, R-spondin 2, R-spondin 3, and R-spondin 4 is an exemplary example. The R-spondin family is a secretory protein and is known to be involved in the activation and control of the Wnt signaling pathway. In the cell culture medium according to the embodiment, a plurality of types of R-spondin may be used in combination.

The R-spondin may be a fragment of the R-spondin or may contain an amino acid sequence other than the amino acid sequence of the R-spondin as long as the R-spondin has an R-spondin activity.

### <<GSK-3β (glycogen synthase kinase-3β) inhibitor>>

As the GSK-3β inhibitor, for example, a FRAT family member that inhibits the interaction between CHIR-99021 (CAS number: 252917-06-9), CHIR-98014 (CAS number: 252935-94-7), lithium, kenpaullone (CAS number: 142273-20-9), 6-bromoindirubin-30-acetoxime, SB216763 (CAS number: 280744-09-4), SB415286 (CAS number: 264218-23-7), or GSK-3 and axin, a FRAT-derived peptide, and the like are exemplary examples.

### <<Afamin>>

Afamin means a glycoprotein belonging to the albumin family and is known to be present in blood or body fluids. In serum that is usually added to culture media, afamin derived from an animal from which the serum has been collected is contained. Since impurities other than afamin are contained in the serum, it is preferable to use afamin alone instead of using the serum.

Where the afamin that is contained in culture media is not particularly limited, and it is possible to use afamin derived from various organisms. Among them, mammal-derived afamin is preferable. As the mammal, the same mammals as described above are exemplary examples. The amino acid sequences of major mammalian afamin and the nucleotide sequences of genes that encode the amino acid sequences can be acquired from a well-known database such as GenBank (R). For example, in GenBank (R), the amino acid sequence of human afamin is registered as AAA21612, the nucleotide sequence of the gene that encodes the amino acid sequence is registered with an accession number of L32140, the amino acid sequence of bovine afamin is registered as DAA28569, and the nucleotide sequence of the gene that encodes the amino acid sequence is registered with an accession number of GJ060968.

The afamin that is contained in the culture medium may be natural afamin that is contained in serum or the like and purified by a well-known method or may be recombinant afamin. The recombinant afamin can be produced by appropriately using a well-known gene recombination technique.

As a method for producing the recombinant afamin, the recombinant afamin can be produced by, for example, inserting DNA that encodes afamin into a well-known expression vector, introducing the obtained expression vector into an appropriate host cell to express recombinant afamin, and purifying the recombinant afamin using a well-known purification method. The recombinant afamin may be affinity-tagged afamin. The affinity tag to be added is not particularly limited and can be appropriately selected from well-known affinity tags and used. As the affinity tag, an affinity tag that is recognized by a specific antibody is preferable, and examples thereof include a FLAG tab, an MYC tag, an HA tag, a V5 tag, and the like.

The above-described Wnt protein has strong hydrophobicity because a specific serine residue is modified with a fatty acid (palmitoleic acid). Therefore, the Wnt protein is easily aggregated or denatured in an aqueous solution, and thus it is widely known that it is extremely difficult to purify and store the Wnt protein.

Incidentally, it has been reported that modification of this specific serine residue with a fatty acid is essential for the physiological activity of the Wnt protein and is involved in binding to the Frizzled receptor family member.

In addition, it is known that the Wnt protein binds to afamin on a one-to-one basis to form a complex in an aqueous solution and is solubilized while maintaining a high physiological activity. The Wnt protein-afamin complex may be produced by a method in which a cell that expresses both the Wnt protein and afamin is cultured, and the Wnt protein-afamin complex may be produced by a method in which a Wnt protein-expressing cell and an afamin-expressing cell are co-cultured.

The concentration of afamin contained in the culture medium is not particularly limited, but is preferably 50 ng/mL or higher and 10 µg/mL or lower, more preferably 100 ng/mL or higher and 1 µg/mL or lower, and still more preferably 300 µg/mL or higher and 1 µg/mL or lower.

### (BMP inhibitor)

BMP binds as a dimeric ligand to a receptor complex consisting of two different receptors serine/threonine kinases: type 1 and type 11 receptors. The type 11 receptor phosphorylates the type I receptor, and, as a result, this receptor kinase is activated. This type I receptor subsequently phosphorylates a specific receptor substrate (SMAD), and, as a result, a transcriptional activity is guided via a signaling pathway. Generally, a BMP inhibitor is an agent that, for example, blocks or inhibits the binding of a BMP molecule to a BMP receptor and a drug that binds to the BMP molecule to form a complex that neutralizes the BMP activity. In addition, the BMP inhibitor is an agent that, for example, binds to a BMP receptor and blocks or inhibits the binding of a BMP molecule to the receptor and a drug that acts as an antagonist or inverse agonist.

The BMP inhibitor has an inhibitory activity of preferably 50% or more, more preferably 70% or more, still more preferably 80% or more, and particularly preferably 90% or more compared to the BMP activity level in the absence of this inhibitor.

The BMP inhibitor is preferably a naturally occurring BMP binding protein, and examples thereof include chordin-like proteins such as noggin, gremlin, chordin, and chordin domains; follistatin-related proteins such as follistatin and follistatin domain; DAN-like proteins such as DAN and DAN cysteine-knot domain; sclerostin/SOST, decorin, α-2 macroglobulin, and the like.

The BMP inhibitor contained in the culture medium is, particularly, preferably a chordin-like protein or a DAN-like protein and more preferably a chordin-like protein. The chordin-like protein is preferably noggin. The chordin-like protein or the DAN-like protein is a diffusible protein and is capable of binding to a BMP molecule with various affinities and inhibiting the access of the BMP molecule to a signaling receptor. In a case where an epithelial stem cell is cultured, the loss of the stem cell can be prevented by adding these BMP inhibitors to the cell culture medium.

The concentration of the BMP inhibitor contained in the culture medium is preferably 10 ng/mL or higher and 100 ng/mL or lower, more preferably 20 ng/mL or higher and 100 ng/mL or lower, and still more preferably 50 ng/mL or higher and 100 ng/mL or lower.

### (TGF-β inhibitor)

TGF-β is a type of growth factor and is produced in almost all cells of kidney, bone marrow, platelets, and the like. As TGF-β, there are five subtypes (β1 to β5). In addition, it is known that TGF-β promotes the growth of osteoblasts and the synthesis and growth of connective tissues such as collagen and acts to suppress the growth of epithelial cells and osteoclasts. Generally, a TGF-β inhibitor is an agent that, for example, blocks or inhibits the binding of TGF-β to a TGF-β receptor and a drug that binds to TGF-β to form a complex that neutralizes the TGF-β activity. In addition, the TGF-β inhibitor is an agent that, for example, binds to a TGF-β receptor and blocks or inhibits the binding of TGF-β to the receptor and a drug that acts as an antagonist or inverse agonist.

As the TGF-β inhibitor, for example, A83-01 (CAS number: 909910-43-6), ALK5 inhibitor I (3-(pyridin-2-yl)-4-(4-quinonyl)-1H-pyrazole), LDN193189 (CAS number: 1062368-24-4), SB-431542 (CAS number: 301836-41-9), SB-505124 (CAS number: 694433-59-5), SD-208 (CAS number: 627536-09-8), SB-525334 (CAS number: 356559-20-1), LY364947 (CAS number: 396129-53-6), LY2157299 (CAS number: 700874-72-2), TGF-β RI kinase inhibitor II 616452 (CAS Number: 446859-33-2), TGF-β R1 kinase inhibitor III 616453 (CAS Number: 356559-13-2), TGF-β RI kinase inhibitor IX 616463 (4-((4-((2,6-dimethylpyridin-3-yl)oxy)pyridin-2-yl)amino)benzenesulfonamide), TGF-β RI kinase inhibitor VII 616458 (CAS Number: 666729-57-3), TGF-β RI kinase inhibitor VIII 616459 (CAS number: 356559-20-1), AP12009 (TGF-β2 antisense compound "Trabedersen"), belagen pumatucel-L (TGF-β2 antisense gene-modified allogeneic tumor cell vaccine), CAT-152 (glaucoma-lerdelimumab (anti-TGF-(3-2 monoclonal antibody)), CAT-192 (metelimumab (human IgG4 monoclonal antibody that neutralizes TGFβ1), GC-1008 (anti-TGF-β monoclonal antibody), and the like are exemplary examples. As the TGF-β inhibitor, among these, A83-01 is preferable.

The concentration of the TGF-β inhibitor contained in the culture medium is preferably 100 nM or higher and 10 µM or lower, more preferably 500 nM or higher and 5 µM or lower, and still more preferably 500 nM or higher and 2 µM or lower.

### (Other components)

The culture medium may further contain a Rho-kinase (Rock) inhibitor. As the Rock inhibitor, for example, Y-27632 (CAS number: 129830-38-2), fasudil (HA1077) (CAS number: 103745-39-7), H-1152 (CAS number: 871543-07-6), and the like are exemplary examples. In a case where Y-27632 is used as the Rock inhibitor, it is preferable to add Y-27632 during the first two days of the culture of stem cells dispersed in a single cell. Y-27632 contained in the culture medium is preferably about 10 µM.

The culture medium may further contain at least one amino acid. As the amino acid, for example, L-alanine, L-arginine, L-asparagine, L-aspartic acid, L-cysteine, L-cystine, L-glutamic acid, L-glutamine, L-glycine, L-histidine, L-isoleucine, L-leucine, L-lysine, L-methionine, L-phenylalanine, L-proline, L-serine, L-threonine, L-tryptophan, L-tyrosine, L-valine, combinations thereof, and the like are exemplary examples. The concentration of L-glutamine contained in the culture medium is 0.05 g/L or higher and 1 g/L or lower (usually 0.1 g/L or higher and 0.75 g/L or lower). Other amino acids contained in the medium are 0.001 g/L or higher and 1 g/L (usually 0.01 g/L or higher and 0.15 g/L or lower). The amino acid may be synthetic amino acid.

The culture medium may further contain at least one vitamin. As the vitamin, for example, thiamine (vitamin B1), riboflavin (vitamin B2), niacin (vitamin B3), calcium D-pantothenate (vitamin B5), pyridoxal/pyridoxamine/pyridoxine (vitamin B6), folic acid (vitamin B9), cyanocobalamin (vitamin B12), ascorbic acid (vitamin C), calciferol (vitamin D2), DL-α tocopherol (vitamin E), biotin (vitamin H), menadione (vitamin K), and the like are exemplary examples.

The culture medium may further contain at least one inorganic salt. The Inorganic salt is contained to help maintain the osmotic balance of cells and to help regulate the membrane potential. Specific examples of the inorganic salt include salts of calcium, copper, iron, magnesium, potassium, sodium, and zinc. The salt is usually used in a form of chloride, phosphate, sulfate, nitrate, or bicarbonate. More specific examples of the salt include CaCl₂, CuSO₄-5H₂O, Fe(NO₃)-9H₂O, FeSO₄-7H₂O, MgCl, MgSO₄, KCl, NaHCO₃, NaCl, Na₂HPO₄, Na₂HPO₄-H₂O, ZnSO₄-7H₂O, and the like.

The culture medium may further contain at least one sugar that can serve as a carbon energy source. As the sugar, for example, glucose, galactose, maltose, fructose, and the like are exemplary examples. Among these, glucose is preferable, and D-glucose (dextrose) is particularly preferable. The concentration of the sugar contained in the culture medium is preferably 1 g/L or higher and 10 g/L.

The culture medium may further contain at least one trace element. As the trace element, for example, barium, bromium, cobalt, iodine, manganese, chromium, copper, nickel, selenium, vanadium, titanium, germanium, molybdenum, silicon, iron, fluorine, silver, rubidium, tin, zirconium, cadmium, zinc, aluminum, ions thereof, and the like are exemplary examples.

The culture medium may further contain at least one additional drug. As such a drug, nutrients or growth factors reported to improve stem cell culture, for example, cholesterol, transferrin, albumin, insulin, progesterone, putrescine, selenite, and the like are exemplary examples.

### [Treatment drug for small cell lung cancer]

In one embodiment, the present invention provides a therapeutic agent for small cell lung cancer, which contains an IGF1R inhibitor as an active ingredient. In the therapeutic agent of this embodiment, it is preferable that the small cell lung cancer is a small cell lung cancer in which the expression level of YAP1 or its downstream factors is significantly higher than that of the control. Downstream factors of YAP1 are the same as those mentioned above, and include CTGF, CYR61, CCND1, and the like.

The effectiveness of administering IGF1R inhibitors in the treatment of patients with cellular lung cancer has been investigated at the basic research level since the early 2000s, but it has been difficult to select appropriate patient subgroups and biomarkers. (For example, G Sakuntala Warshamana-Greene, et al., The insulin-like growth factor-I receptor kinase inhibitor, NVP-ADW742, sensitizes small cell lung cancer cell lines to the effects of chemotherapy, Clin Cancer Res., 11 (4), 1563-1571, 2005.; Rebekah L Zinn, et al., ERK phosphorylation is predictive of resistance to IGF-1R inhibition in small cell lung cancer, Mol Cancer Then, 12 (6), 1131-1139, 2013. ).

In contrast, as described later in the Examples, the inventors found that small cell lung cancer cells that express high levels of YAP1 or its downstream factors have suppressed proliferation and induced apoptosis in the presence of an 1GF1R inhibitor. revealed. Therefore, IGF1R inhibitors are effective in treating small cell lung cancer patients with high expression levels of YAP1 or its downstream factors.

The same applies to small cell lung cancer in which the expression level of YAP1 or its downstream factors is significantly higher than the control. Furthermore, the IGF1R inhibitors are the same as those described above.

The therapeutic agent of this embodiment may be formulated as a pharmaceutical composition for treating small cell lung cancer in which the expression level of YAP1 or its downstream factors is high. Pharmaceutical compositions can be administered orally in the form of tablets, capsules, elixirs, microcapsules, etc., or parenterally in the form of injections, suppositories, skin external preparations, etc.. More specifically, the external skin preparation includes dosage forms such as ointments and patches.

As the pharmaceutically acceptable carrier, those commonly used in the formulation of pharmaceutical compositions can be used. More specifically, for example, binders such as hypromellose, dextrin, macrogol 400, gelatin, corn starch, gum tragacanth, and gum arabic; excipients such as lactose hydrate, D-mannitol, starch, crystalline cellulose, and alginic acid. Injectable solvents such as water, ethanol and glycerin; Adhesives such as rubber adhesives and silicone adhesives.

The therapeutic agent of this embodiment may contain additives. Additives include lubricants such as calcium stearate and magnesium stearate; sweeteners such as sucrose, lactose, saccharin, and maltitol; flavoring agents such as peppermint and red oil; carmellose sodium, hydrogenated oil, and light anhydrous silicic acid. Stabilizers such as , povidone, glycerin fatty acid ester, benzyl alcohol, phenol; Buffers such as phosphates, sodium acetate; Solubilizing agents such as benzyl benzoate, benzyl alcohol; Yellow iron sesquioxide, iron sesquioxide, black oxide Examples include coloring agents such as iron and titanium oxide.

A pharmaceutical composition for treating small cell lung cancer in which the expression level of YAP1 or its downstream factors is high can be obtained by appropriately combining the above-mentioned IGF1R inhibitor and the above-mentioned pharmaceutically acceptable carriers and additives, according to generally accepted pharmaceutical practices. It can be formulated by mixing in unit dosage forms as required. In the pharmaceutical composition, one type of IGF1R inhibitor may be used alone, or two or more types may be used in combination.

The appropriate daily dose of the therapeutic agent or pharmaceutical composition for small cell lung cancer in which the expression level of YAP1 or its downstream factors is high is the lowest dose of the active ingredient (IGF1R inhibitor) effective to produce a therapeutic effect. This is the amount including. The minimum effective dose depends on the activity of the active ingredient contained in the pharmaceutical composition, functional group modification that defines fat solubility/water solubility, administration route, administration time, excretion rate of the specific active ingredient used, treatment period, It depends on a variety of factors, including other drugs used, age, sex, weight, disease, health condition, medical history of the patient, and other factors well known in the medical arts.

Usually, the dosage of a therapeutic agent or pharmaceutical composition for patients with small cell lung cancer who express high levels of YAP1 or its downstream factors is about 0.0001 to about 100 mg/kg body weight of the active ingredient (IGF1R inhibitor) per day. This is the amount included. The therapeutic agent or pharmaceutical composition may be administered once a day or divided into about 2 to 4 times a day.

[Method of determining whether administration of an IGF1R inhibitor is effective in treating patients with small cell lung cancer] In one embodiment, the present invention provides a method for determining whether administration of an IGF1R inhibitor is effective in treating patients with small cell lung cancer. The method includes the step of detecting the expression of YAP1 or its downstream factors in the cancer cells derived from the small cell lung cancer patient, and the method includes the step of detecting the expression of YAP1 or its downstream factors in the cancer cells derived from the small cell lung cancer patient, and the expression level of the YAP1 or its downstream factors is compared with that of a control. The present invention provides a method in which the administration of an IGF1R inhibitor is effective in treating the small cell lung cancer patient.

Examples of cancer cells derived from patients with small cell lung cancer include cancer cells derived from cancer tissues of patients with small cell lung cancer, cancer cells derived from pleural effusion, cancer cells derived from sputum, and cancer cells circulating in the blood. Furthermore, organoids prepared from these cancer cells can also be used. An organoid is a three-dimensional cell tissue that is self-organized by an accumulation of cells, and has a structure and function similar to an organ in a living body.

The NCBI accession numbers of the cDNA of the human YAP1 gene are NM_001130145.3, NM_001195044.2, NM_001195045.2, and the like.

A downstream factor of YAP1 include CTGF, CYR61, CCND1, and the like. The NCBI accession number of the cDNA of the human CTGF gene is NM_001901.4, and the like. Furthermore, the NCBI accession number of the cDNA of the human CYR61 gene is NM_001554.5. The NCBI accession number of the cDNA of the human CCND1 gene is NM_053056.3.

As used herein, expression of YAP1 or its downstream factors in cancer cells that is significantly higher than that of a control means expression of YAP1, CTGF, CYR61, CCND1, etc. at the gene level or protein level in cancer cells, means that the amount is significantly higher compared to the control. Significantly high may mean statistically significantly high.

Here, examples of controls include IGF-independent small cell lung cancer cells, ASCL1-positive small cell lung cancer cells, NEUROD1-positive small cell lung cancer cells, and the like.

The NCBI accession number of the cDNA of the human ASCL1 gene is NM_004316.4, etc. Furthermore, the NCBI accession number of the cDNA of the human NEUROD1 gene is NM_002500.5, etc. Further, the NCBI accession numbers of the cDNA of the human POU2F3 gene are NM_001244682.2, NM_014352.4, etc.

In the method of this embodiment, the expression of YAP1 may be detected by immunostaining of YAP1 protein. Here, any antibody that recognizes YAP1 can be used as the antibody (primary antibody) for immunostaining. Moreover, any secondary antibody that recognizes the above-mentioned primary antibody can be used as the secondary antibody.

It was said that immunostaining for YAP1 usually does not work well. In contrast, the inventors have found that YAP1 immunostaining can be successfully performed by performing immunostaining under the conditions and method described in the Examples.

An 1GF1R inhibitor is a substance that inhibits downstream signal transduction of IGF1R. The NCBI accession numbers of the cDNA of the human IGF1R gene are NM_000875.5, NM_001291858.2, NM_152452.1, etc.

Specific IGF1R inhibitors include linsitinib (CAS number: 867160-71-2), BMS-754807 (CAS number: 1001350-96-4), BVP-51004 (CAS number: 477-47-4), XL -228 (CAS number: 898280-07-4), anti-IGF1R antibody, and the like.

### [Immunostaining method for YAP1 protein]

In one embodiment, the present invention provides a method for immunostaining YAP1 protein in a biological sample, in which an anti-YAP1 rabbit monoclonal antibody is used as the primary antibody, and an anti-rabbit IgG antibody is used as the secondary antibody at a ratio of 1:50 to 1:50. A method is provided, including using a 1:500 dilution. The dilution of the antibody can be changed as appropriate depending on the characteristics of the secondary antibody, and does not need to be at the above ratio.

The biological sample is not particularly limited, and examples include thin sections of cancer tissues and organoids. It was said that YAP1 immunostaining usually does not work well. In contrast, the inventors have found that YAP1 immunostaining can be performed satisfactorily by performing immunostaining under the above conditions.

The dilution ratio of the anti-rabbit IgG antibody can be determined as appropriate depending on the characteristics of the antibody used.

### [Other embodiments]

In one embodiment, the present invention provides a step of detecting the expression of YAP1 or a downstream factor thereof in cancer cells derived from a small cell lung cancer patient, and a step of detecting the expression of YAP1 or a downstream factor thereof in cancer cells derived from a patient with small cell lung cancer, and and administering an effective amount of an IGF1R inhibitor to the small cell lung cancer patient when the IGF1R inhibitor is high.

In one embodiment, the present invention provides a method for treating small cell lung cancer in which the expression level of YAP1 or its downstream factors is significantly higher compared to a control, including administering an effective amount of an IGF1R inhibitor to a patient in need of treatment.

In one embodiment, the present invention provides an 1GF1R inhibitor for use in the treatment of small cell lung cancer in which the expression level of YAP1 or its downstream factors is significantly higher compared to a control.

In one embodiment, the present invention provides the use of an IGF1R inhibitor for producing a therapeutic agent for small cell lung cancer in which the expression level of YAP1 or its downstream factors is significantly higher than that of a control.

In each of these embodiments, YAP1 or its downstream factors, small cell lung cancer in which the expression level of YAP 1 or its downstream factors is significantly higher than the control, and the 1GF1R inhibitor are the same as those described above.

### [Examples]

Next, the present invention will be described in more detail by showing examples, but the present invention is not limited to the following examples.

### [Experiment Example 1]

### (Establishment of small cell lung cancer organoid library)

Small cell lung cancer samples were collected from surgical resection samples, endoscopic biopsy samples, circulating tumor cells (CTCs), sputum, or pleural effusions.

Small cell lung cancer organoids from surgical resection samples, endoscopic biopsy samples, sputum and pleural effusion samples were prepared as recited in Kawasaki K., et al., An Organoid Biobank of Neuroendocrine Neoplasms Enables Genotype-Phenotype Mapping, Cell, 183 (5). , 1420-1435, 2020.

Separation of CTC was performed as follows. First, 15 mL of blood sample was placed into a heparin tube. Then, 750 µL of RosseteSep Human CD36 Depletion Cocktail (Stem Cell Technology) was added to the blood and incubated for 20 minutes. Subsequently, red blood cells and white blood cells were removed by centrifugation at 1200×g for 20 minutes using Ficoll-Paque PLUS (Cytiva) to collect CTCs.

Subsequently, the collected CTCs were embedded in droplets of Matrigel (registered trademark), and overlaid with complete medium to culture. The complete medium was prepared by adding a niche factor whose composition is shown in Table 2 below to a basic culture medium whose composition is shown in Table 1 below. As a result, organoids were established from CTCs.

**[Table 1]**

| Basic Culture Medium | |
|---|---|
| Component | Concentration |
| Advanced DMEM/F12 | - |
| HEPES | 10 mM |
| Penicillin-Streptomycin | 1% |
| GrutaMAX | 2 mM |
| B-27 supplement (Thermo Fisher Scientific) | 1X |
| N-Acetyl-L-cysteine (FUJIFILM Wako Pure Chemical Corporation) | 1mM |

**[Table 2]**

| Niche Factors | |
|---|---|
| Component | Concentration |
| Afamin-Wnt-3A conditioned medium | 10% |
| Recombinant mouse EGF (Thermo Fisher Scientific) | 50 ng/mL |
| Recombinant human FGF-2 (PeproTech) | 50 ng/mL |
| Recombinant human IGF-1 (BioLegend) | 100 ng/mL |
| Recombinant human R-spondin1 (R&D) | 5% |
| Recombinant mouse Noggin (PeproTech) | 5% |
| A83-01 (Tocris) | 500 nM |
| Y-27632 (FUJIFILM Wako Pure Chemical Corporation) | 10 µM |

41 organoid lines were established from cancer cells derived from 28 small cell lung cancer patients to obtain a small cell lung cancer organoid library.

### [Experiment Example 2]

### (RNA-seq analysis of small cell lung cancer organoid library)

Small cell lung cancer organoids were dissociated into a single cell and cultured for 7-14 days in the complete medium described above. Subsequently, RNA was extracted from each organoid using RNeasy Plus Mini Kit (Qiagen).

Subsequently, the RNA quality was evaluated with an Agilent 2100 bioanalyzer (Agilent). Subsequently, a sequencing library was prepared with TruSeq RNA Library Prep Kit v2 (Illumina) and sequenced using HiSeq 4000 (Illumina) or NovaSeq 6000 (Illumina).

Adaptor sequences were removed from raw fastq files with cutadapt (version 1.18). Subsequently, the reads were aligned to reference human genome sequence hg38 using STAR (version 2.6.1).

Gene sequence analysis was performed using R Bioconductor package DESeq2. The read count matrices were normalized with a size factor, and variance stabilization transformations were performed using the vst function of DESeq2.

Cluster analysis was performed using the R Bioconductor package Consensus Cluster Plus based on Euclidean distance and PAM algorithm. In the cluster analysis, genes with a small variance, namely, the standard deviation of log2-normalized expression values <1.5, were discarded.

In addition, differentially expressed genes were identified using the nbinomLRT function in DESeq2.

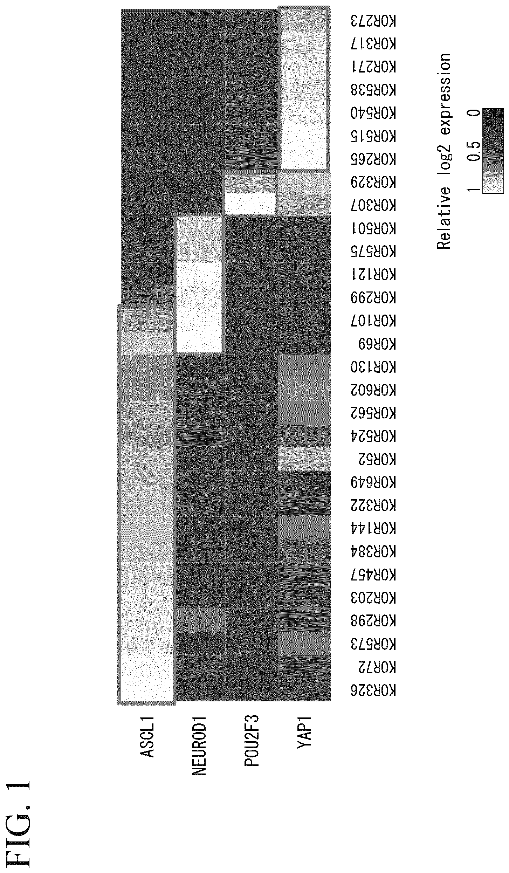

Figure 1 is a heat map showing the expression of respective transcription factors, ASCL1, NEUROD1, POU2F3, and YAP1, in respective small cell lung cancer organoid based on the results of RNA-seq analysis. In FIG. 1, the horizontal axis indicates respective small cell lung cancer organoid lines. The result revealed that respective small cell lung cancer organoids can be classified into four subgroups based on the expression of these transcription factors.

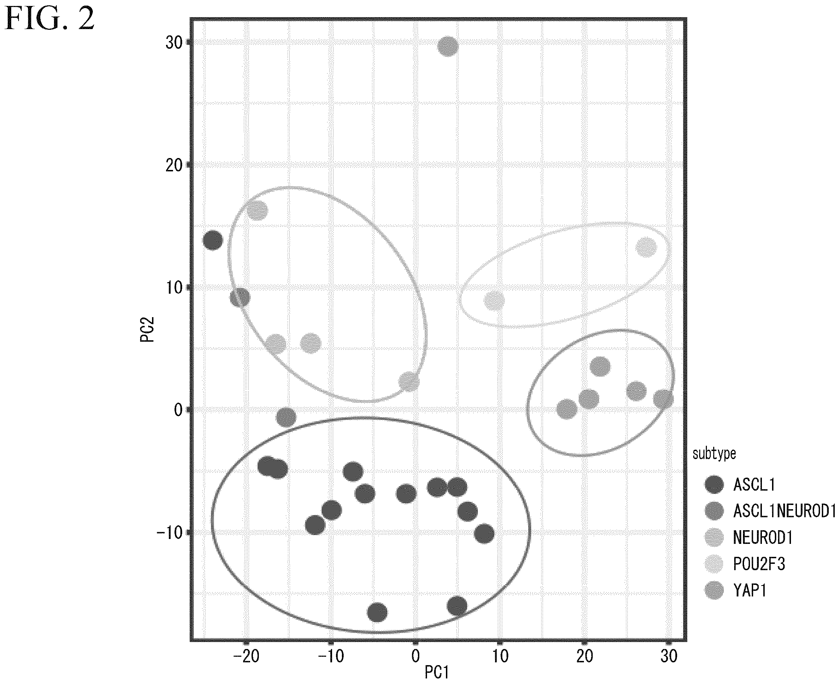

FIG. 2 is a graph showing the results of principal component analysis based on the results of RNA-seq analysis. The result revealed that respective small cell lung cancer organoids can be classified into four subgroups based on difference of expression profiles.

### [Experiment Example 3]

### (Study of IGF dependence of small cell lung cancer organoids)

Small cell lung cancer organoids were dissociated into single cells using TrypLE Express (Thermo Fisher Scientific), and further filtered using a 20 µm cell strainer.

Subsequently, 100 cells were seeded per well and cultured under different medium conditions. The above-mentioned complete medium (Complete), a medium from which EGF, IGF1 and FGF2 are removed from the complete medium (-EIF), a medium from which IGF1 is removed from the complete medium (-1GF1), and a medium from which EGF is removed from the complete medium (-EGF) and a medium from which FGF2 is removed from the complete medium (-FGF2) were used as mediums. Two days after seeding, 10 µM Y-27632 was added to the mediums. Subsequently, 14 to 21 days after seeding, images of the cells were taken using a BZX-710 digital microscope (Keyence).

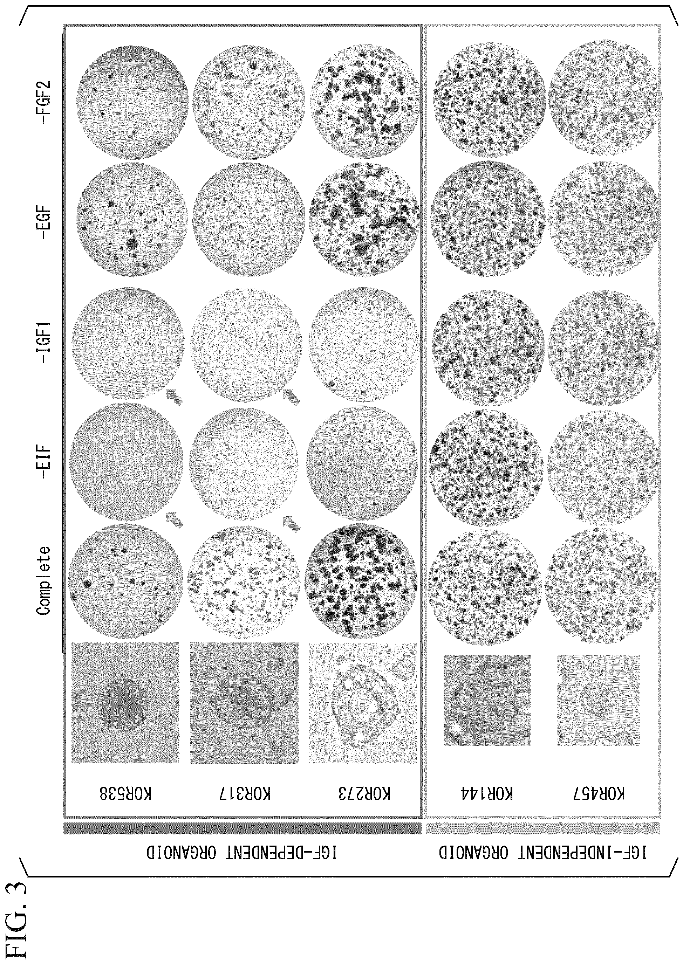

Figure 3 is a representative microphotograph of small cell lung cancer organoids. As a result, it was revealed that there is a subgroup of small cell lung cancer organoids that is specifically dependent on 1GF1. In FIG. 3, arrows indicate examples in which organoid proliferation is particularly suppressed.

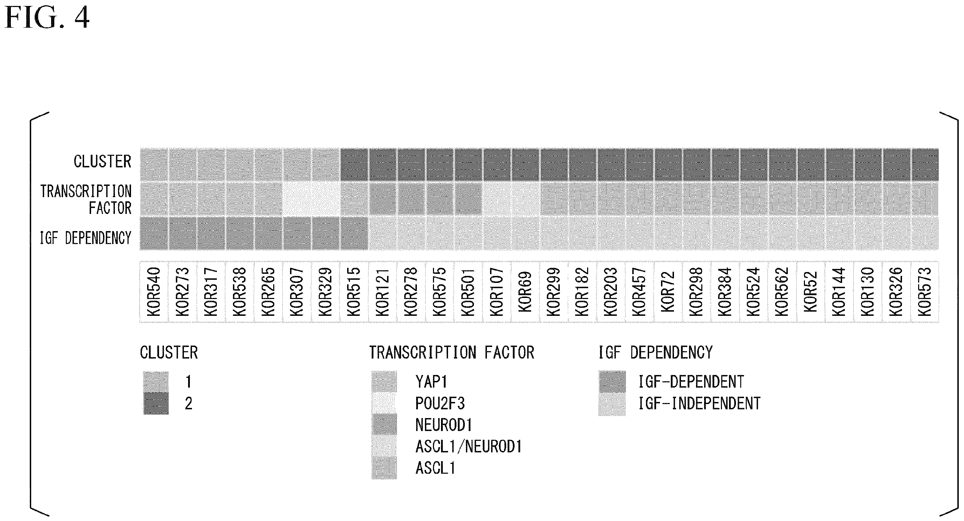

FIG. 4 is a diagram showing the relationship between IGF dependency, kinds of expressing transcription factors, and clusters for respective small cell lung cancer organoid lines. In FIG. 4, the horizontal axis indicates respective small cell lung cancer organoid lines. As a result, it was revealed that the YAP1-positive small cell lung cancer organoid lines were IGF-dependent.

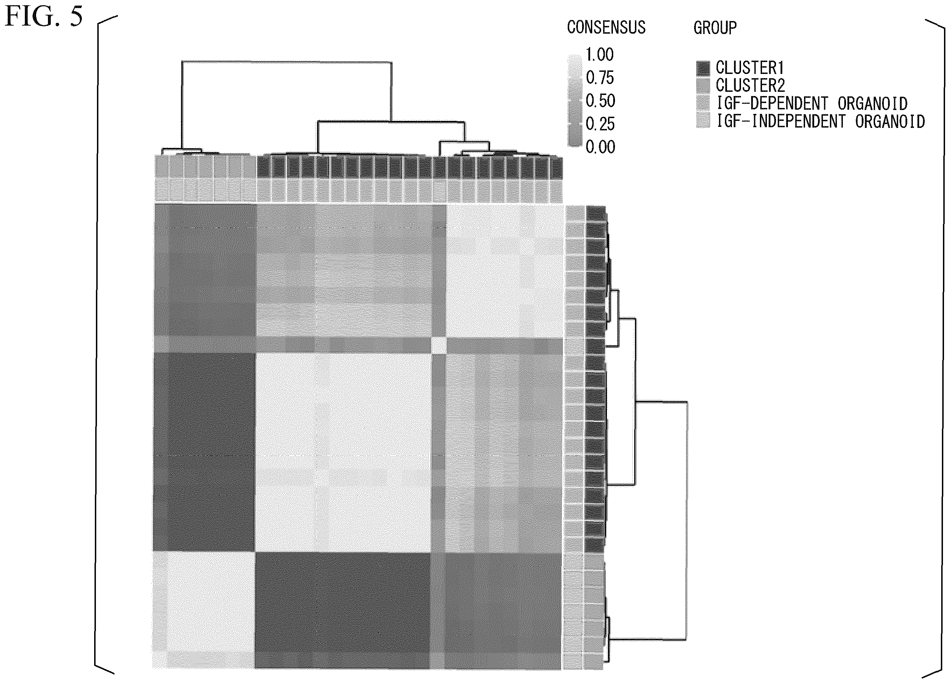

FIG. 5 is a diagram showing the results of co-expression cluster analysis based on the results of RNA-seq analysis. The results revealed that the expression profiles of IGF-dependent organoids and IGF-independent organoids were different.

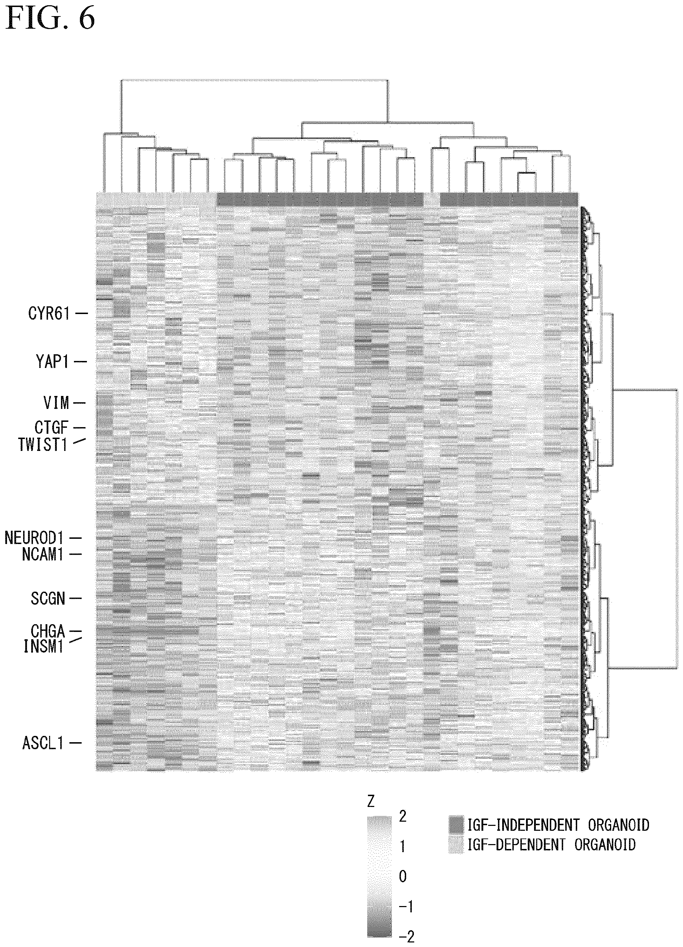

Furthermore, FIG. 6 is a diagram showing the results of hierarchical cluster analysis based on the results of RNA-seq analysis. As a result, it was revealed that IGF-dependent organoids characteristically express YAP1 and its downstream factors such as CTGF and CYR61.

### [Experiment Example 4]

### (Immunostaining of small cell lung cancer organoids)

Small cell lung cancer organoids were immunostained to detect the expression of YAP1. The organoids were fixed after jellifying with iPGell (Genostaff), and thin sliced sections were prepared. As a primary antibody, an anti-YAP1 rabbit monoclonal antibody (product name "D8H1X", catalog number "#14074", Cell Signaling Technology) diluted 1:400 was used. In addition, as a secondary antibody, Bond^{™} Anti-rabbit Poly-HRP-IgG from the Polymer Refine Detection Kit (catalog number "DS9800", Leica Biosystems) was diluted 1:100 to be used.

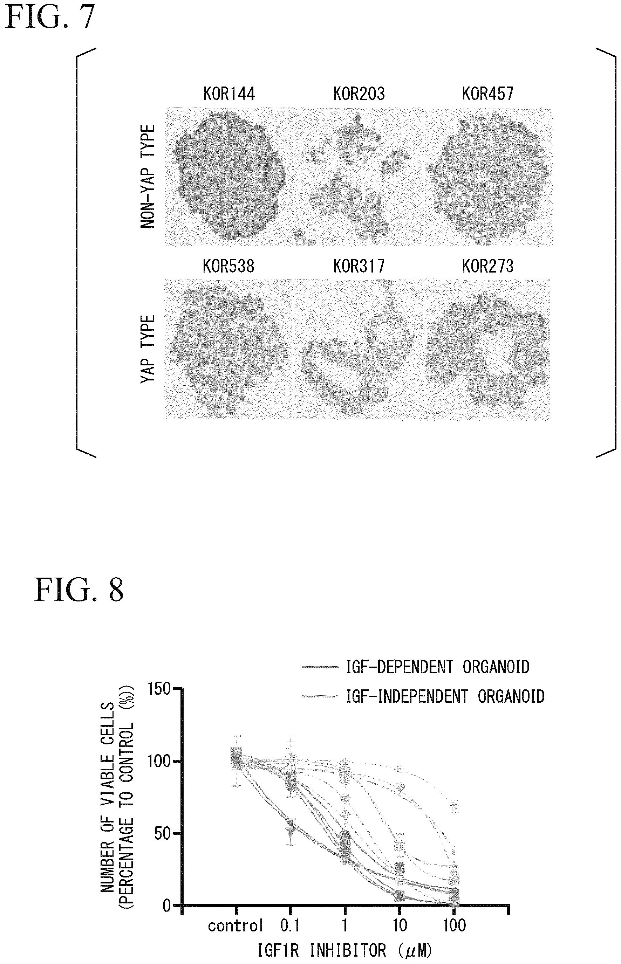

Figure 7 is a photograph showing the results of immunostaining. Staining with anti-YAP1 antibody was observed in IGF1-dependent small cell lung cancer organoid lines KOR538, KOR317, and KOR273, but no staining was observed in IGF1-independent small cell lung cancer organoid lines KOR144, KOR203, and KOR457. This result confirmed that the expression of YAP1 could be detected by immunostaining.

### [Experiment Example 5]

### (Effect of IGF1R inhibitor in vitro)

An IGF1R inhibitor was added to the medium of small cell lung cancer organoids, and the effect on the growth of small cell lung cancer organoids was investigated. As small cell lung cancer organoids, a plurality of types of each of IGF-dependent small cell lung cancer organoid lines and IGF-independent small cell lung cancer organoid lines were used. Furthermore, linsitinib (CAS number: 867160-71-2) was used as an IGF1R inhibitor. In addition, the number of living cells was measured using a commercially available kit (product name "CellTiter-Glo", Promega).

A small cell lung cancer organoid was dissociated into a single cell using TrypLE Express (Thermo Fisher Scientific), and further filtered using a 20 µm cell strainer.

Then, 3 × 10³ per well cells were seeded and cultured in complete medium added with 0.1, 1, 10, and 100 µM lincitinib. In addition, as a control, a group in which dimethyl sulfoxide (DMSO), which was used as a solvent for linsitinib, was added was also prepared. The number of living cells was measured on the fifth day after adding the drug.

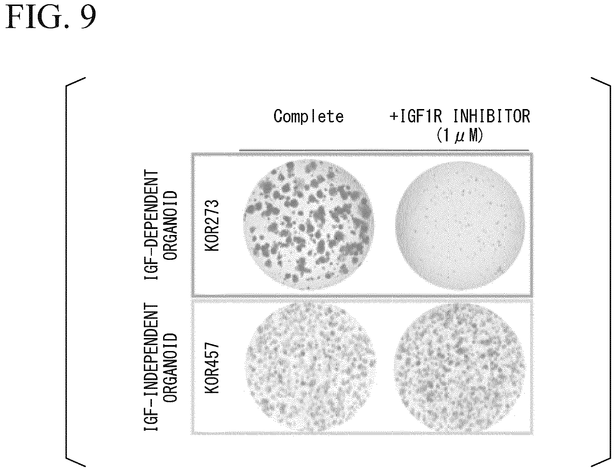

FIG. 8 is a graph showing the measurement results of the number of living cells. As a result, it was revealed that addition of an IGF1R inhibitor to the medium inhibits the growth of IGF-dependent organoids. Figure 9 is a representative photograph of small cell lung cancer organoids 5 days after addition of 1 µM linsitinib.

### [Experiment Example 6]

### (In vitro apoptosis assay)

An IGF1R inhibitor was added to the medium of small cell lung cancer organoids, and the occurrence of apoptosis was measured.

First, a small cell lung cancer organoid was dissociated into a single cell using TrypLE Express (Thermo Fisher Scientific), further, filtered using a 20 µm cell strainer.

Next, add 5 × 10⁴ cells per well were seeded to a 3-well plate, and cultured for 5 days in the presence of 1 µM IGF1R inhibitor or 1 µM FGFR inhibitor. DMSO was added to the control cell culture medium.

Subsequently, using a commercially available kit (product name "TACS Annexin V-FITC Apoptosis Detection Kit", R&D System), the induction of apoptosis was measured by flow cytometry using the BD FACSCalibur system (Becton Dickinson).

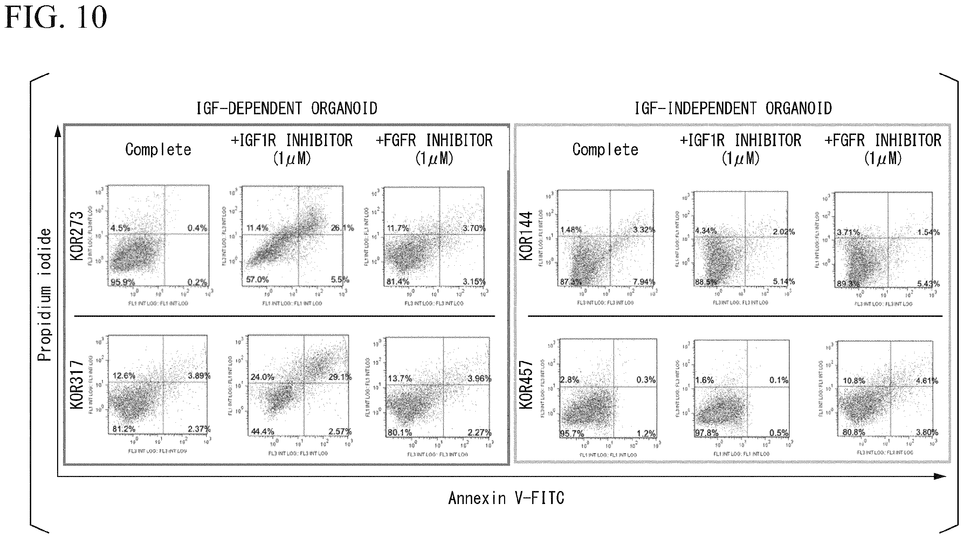

FIG. 10 is a graph showing the results of the flow cytometry. In FIG. 10, the propidium iodide-positive and Annexin V-positive cells are the cells in which apoptosis was induced.

The results revealed that administration of an IGF1R inhibitor to the culture medium induces apoptosis in IGF-dependent organoids.

### [Experiment Example 7]

### (Effect of IGF1R inhibitor in vivo)

An IGF1R inhibitor was administered to tumor-bearing mice formed by transplanting KOR317 line (YAP1 positive subtype) which is an organoid derived from IGF-dependent small cell lung cancer cells, and KOR457 strain (ASCL1 positive subtype) which is an organoid derived from an IGF-independent small cell lung cancer cell, and changes in tumor volume over time were observed.

Specifically, first, small cell lung cancer organoids (KOR317 line and KOR457 line) were peeled off from Matrigel (registered trademark) using cell recovery solution (BD Bioscience). The peeled small cell lung cancer organoids were kept on ice for 30 minutes while being loosened by pipetting every 10 minutes. Next, approximately 1×10 ⁵ of respective small cell lung cancer organoids was resuspended in about 50 µL of Matrigel, and the respective suspensions was subcutaneously injected into mice. As the mice, 6-10 weeks old NOD/Shi-scid, IL-2Ry mice were used.

Once the tumor diameter (long × short × height/2) was approximately 100-150 mm³, the subjects were randomly divided into a drug administration group and a placebo treatment group. Each group was set as n ≧ 3.

Subsequently, linsitinib (50 mg/kg) was administered to the drug administration group, and vehicle was administered to the placebo treatment group once a day, 5 days a week, for a total of 5 weeks. Linsitinib was dissolved in 30% PEG400-0.5% tween80-5% propylene glycol (vehicle) and administered. Tumor diameter (major axis x short axis x height/2) was measured once a week.

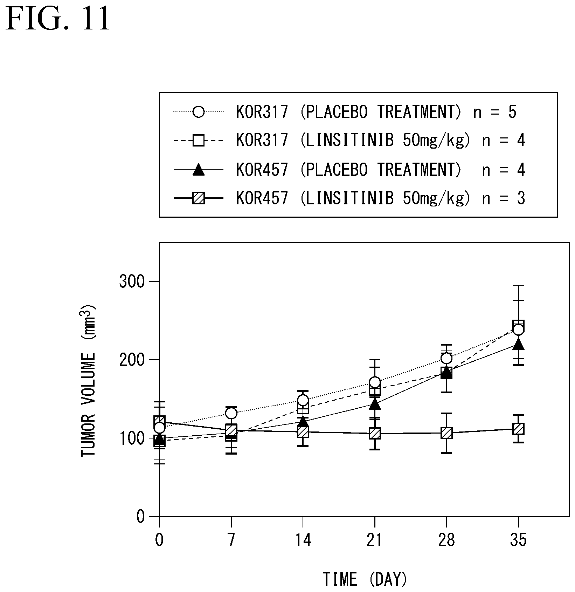

FIG. 11 is a graph showing the results of measuring tumor volume over time after the start of drug or vehicle administration. As a result, as shown in FIG. 11, tumor growth was suppressed in the group in which an IGF1R inhibitor was administered to mice transplanted with KOR317 line which isYAP1-positive, namely IGF-dependent. On the other hand, no effect of the IGF1R inhibitor was observed in mice transplanted with the KOR457 line which is ASCL1-positive. This revealed that the IGF1R inhibitor is a specific therapeutic agent for YAP1-positive small cell lung cancer.

### [Industrial applicability]

According to the present invention, a technique is provided for identifying a subgroup of small cell lung cancer for which administration of a molecular target therapeutic agent is effective, clarifying the molecular target therapeutic agent, and determining the effectiveness of administration of the molecular target therapeutic agent.

## Claims

1. A therapeutic agent for small cell lung cancer, which comprises an insulin-like growth factor 1 receptor (IGF1R) inhibitor as an active ingredient.

2. The therapeutic agent according to Claim 1, wherein the small cell lung cancer is a small cell lung cancer in which an expression level of Yes1 Associated Transcriptional Regulator (YAP1) or an expression level of a downstream factor of YAP1 is significantly higher than that of a control.

3. The therapeutic agent according to Claim 2, wherein the downstream factor of YAP1 is connected tissue growth factor (CTGF), cysteine-rich angiogenic inducer 61 (CYR61), or Cyclin D1 (CCND1).

4. A method for determining whether or not an administration of the therapeutic agent according to any one of Claims 1 to 3 is effective in treating a small cell lung cancer patient, comprising:
a step of detecting an expression of YAP1 or an expression level of a downstream factor of YAP1 in a cancer cell from the small cell lung cancer patient,
wherein when the expression level of YAP1 or the expression level of the downstream factor of YAP1 is significantly higher than that of a control, it is shown that the administration of the therapeutic agent according to any one of Claims 1 to 3 is effective.

5. The method according to Claim 4, wherein the downstream factor of YAP1 is CTGF, CYR61 or CCND1.

6. The method according to Claim 4, wherein the detecting of the expression of YAP1 is performed by immunostaining of YAP1 protein.

7. The method according to any one of Claims 4 to 6, wherein the cancer cell from the small cell lung cancer patient is a small cell lung cancer cell-derived organoid.

8. A method for producing a small cell lung cancer cell-derived organoid, comprising a step of culturing a cancer cell from a small cell lung cancer patient in a medium.

9. The method for producing according to Claim 8, wherein the medium comprises:
at least two selected from the group consisting of insulin-like growth factor 1 (IGF1), fibroblast growth factor 2 (FGF2), EGF (Epidermal Growth Factor) and epiregulin (EREG);
and at least one selected from the group consisting of Wnt agonists, bone morphogenetic protein (BMP) inhibitors, and transforming growth factor-β (TGF-β) inhibitors.

10. The method for producing according to Claim 8 or 9, wherein the cancer cell is a cancer tissue-derived cancer cell, a pleural effusion-derived cancer cell, a sputum-derived cancer cell, or a circulating cancer cell.
